# EUROPÄISCHE PATENTSCHRIFT

(11) **EP 1 466 171 B1**
(45) Veröffentlichungstag und Bekanntmachung des Hinweises auf die Patenterteilung: **17.05.2006**
(21) Anmeldenummer: 02762199.4
(22) Anmeldetag: 23.09.2002
(51) Int. Cl.: G01N 33/18, G01N 17/00

(54) **VERFAHREN ZUR VERMINDERUNG VON SCHÄDEN AN HEIZUNGSANLAGEN UND VORRICHTUNG ZUR DURCHFÜHRUNG DES VERFAHRENS**
METHOD FOR REDUCING DAMAGE TO HEATING PLANTS AND DEVICE FOR CARRYING OUT SAID METHOD
PROCEDE POUR REDUIRE LES DETERIORATIONS AU NIVEAU D'INSTALLATIONS DE CHAUFFAGE ET DISPOSITIF POUR METTRE LEDIT PROCEDE EN OEUVRE

(30) Priorität: 21.09.2001 CH 1810927
(43) Veröffentlichungstag der Anmeldung: 13.10.2004
(60) Teilanmeldung: 05013646.4
(73) Patentinhaber: Swiss E-Technic AG, 7303 Mastrils (CH)
(72) Erfinder: FÜLLEMANN, Jörg, CH-7303 Mastrils (CH)
(74) Vertreter: Walder, Martin Bernhard
(86) Internationale Anmeldenummer: PCT/CH2002/000532
(87) Internationale Veröffentlichungsnummer: WO 2003/027668

(56) Entgegenhaltungen:
- EP-A- 0 761 863
- EP-A- 1 188 989
- US-A- 5 332 961
- US-A- 5 641 841
- PATENT ABSTRACTS OF JAPAN vol. 006, no. 112 (P-124), 23. Juni 1982 (1982-06-23) & JP 57 040652 A (MITSUBISHI ELECTRIC CORP), 6. März 1982 (1982-03-06)

## Beschreibung

In jüngster Zeit sind schwefelarme Heizöle auf den Markt gekommen, die einen Massenanteil an Schwefel von 0,05 bis 0,001 % aufweisen. Bei der Verwendung solcher schwefelarmer Heizöle treten bisher unbekannte Schadensbilder an den Metallteilen im Kesselraum und am Ölbrenner, insbesondere am Flammbecher auf. Die Schadensbilder treten auch in Brennwert-Heizanlagen auf, die die Kondensationswärme nutzen und mit Niedertemperatur-Brennern, sog. Gebläse-Blaubrennern ausgerüstet sind. Die Schadensbilder zeigen scharf umrissene, kraterförmig erodierte Stellen mit einer fein gekörnten Oberfläche und unterscheiden sich deutlich von einer Hochtemperaturkorrosion. Derartige Schadensbilder können bereits innerhalb weniger Wochen nach Inbetriebnahme der Heizungsanlage entstehen. Die Wahl von korrosionsbeständigeren Materialien hat nur bedingt Einfluss auf die Schadensbilder.

Es ist daher Aufgabe der Erfindung Mittel und Wege zu finden, die neuartigen Schadensbilder an Heizungsanlagen bei der Verwendung von Heizöl mit einem Schwefelgehalte von 0,05 und weniger zu verhindern oder zu vermindern.

Diese Aufgabe wird durch das Verfahren gemäß Anspruch 1 sowie mittels der Vorrichtung gemäß Anspruch 26 gelöst.

In einer Versuchsreihe mit sieben Brennstoffmarken unterschiedlicher Herkunft konnte festgestellt werden, dass die Herkunft des Brennstoffs entscheidenden Einfluss hat auf seine erosive Wirkung. Heizöle mit identischem Schwefelgehalt, jedoch unterschiedlicher Herkunft zeigten unterschiedliche Erosivität. Vergleiche der chemischen und physikalischen Analysen der unterschiedlichen Brennstoffe ergaben bisher keine schlüssigen Resultate über die Ursache der unterschiedlichen Erosivität. Es wird vermutet, dass der Raffinerieprozess oder die Ölquelle die Heizölqualität derart beeinflusst, dass eine Erosion auftritt oder nicht auftritt.

Es wird daher vorgeschlagen, zur Vermeidung der Erosionsschäden in Heizungsanlagen den flüssigen, schwefelarmen Brennstoff, z.B. chargenweise durch eine neutrale Stelle oder in der Raffinerie, auf seine potentielle erosive Wirkung beim Verbrennen in Heizungsanlagen zu prüfen. Zur Prüfung des Brennstoffes wird vorteilhaft der Brennstoff in Verbrennungsluft nahe einer Prüffläche vernebelt und der Brennstoffnebel einem elektrischen Spannungsfeld ausgesetzt und entflammt.

Weshalb dabei an metallischen Prüfflächen eine von blossem Auge sichtbare Erosion bereits nach kurzer Zeit auftritt, ist im Detail nicht bekannt. Es kann dies jedoch folgendermassen erklärt werden: Bekanntlich entsteht bei der Verbrennung von schwefelarmem Heizöl in der Dampf/Flüssigphase und bei Anwesenheit von Sauerstoff Carbonsäure. Es wird vermutet, dass diese Carbonsäure die Metalloxidschicht (überwiegend Eisenoxid), die das Metall schützt, angreift und zerstört und an diesen Stellen ohne Metalloxidschicht das blanke Metall erodiert wird. Es wird weiter angenommen, dass bei der Zündung solch erosiven Heizöls in Gegenwart des elektrischen Spannungsfeldes des Zündfunkens Ionen des Plasmas, gleich anschliessend an deren Entstehung, gegen die Metallfläche schiessen und bei ihrem Aufprall diese erodieren.

Es konnte festgestellt werden, dass bei einer mit einer Kohlenschicht versehenen Prüffläche Schadensbilder rascher auftreten als bei blanken Prüfflächen. Scheinbar wird die Bildung von Carbonsäure durch die Anwesenheit einer Kohlenschicht auf der metallischen Oberfläche begünstigt. Die Kohlenschicht braucht dabei lediglich einige µ stark zu sein. Eine ausreichende Schichtstärke wird auf 10 bis 15 µ geschätzt.

Es konnte ferner festgestellt werden, dass das Durchfahren eines Temperaturbereiches notwendig ist. Dieser Temperaturbereich liegt sicher zwischen 350 und 600 °C. Ein eingeschränkter Temperaturbereich liegt zwischen 370 und 550 °C. In diesem Temperaturbereich treten die Prozesse auf, die das Schadensbild verursachen. Aufgrund von weiteren Untersuchungen ist dieser Temperaturbereich möglicherweise noch präziser einschränkbar. Es konnte weiter beobachtet werden, dass die Schadensbilder beschleunigt an Stellen auftreten, die durch den Brennstoff benetzt werden.

Ein Prüfungsverfahren zur Prüfung von flüssigem Brennstoff auf dessen potentielle erosive Wirkung beim Verbrennen in Heizungsanlagen zeichnet sich vorteilhaft durch eine mehrfache Wiederholung einer Sequenz von Verfahrensschritten aus, welche Sequenz folgende Merkmale aufweist:
- Vernebeln von Brennstoff und vermischen von Brennstoffnebel und Verbrennungsluft,
- ein elektrisches Spannungsfeld errichten und den Brennstoffnebel dem Spannungsfeld aussetzen.
- Zünden des versprühten Brennstoffes, so dass ein Plasma entsteht,
- Aufrechterhalten des elektrischen Spannungsfelds und das Plasma dem elektrischen Spannungsfeld aussetzen,
- Abstellen der Brennstoffzufuhr.

Dabei wird vorteilhaft beim Starten des Prüfverfahrens Heizöl verwendet, das eine zum Starten geeignete Temperatur aufweist. Sobald der Verbrennungsprozess Hitze entwickelt, wird vorteilhaft auf gekühltes Heizöl umgestellt. Das gekühlte Heizöl kann vorteilhaft mit einem Fliessverbesserer versetzt sein, damit es möglichst kühl, z.B. bei minus 10 °C verwendet werden kann. Dank einem gekühlten Brennstoff wird die Prüffläche möglichst lange benetzt.

Obige Sequenz kann vorteilhaft mit einem weiteren Verfahrensschritt ergänzt werden, in welchem vorhandene Prüfflächen gekühlt werden. Zum Kühlen nach Abstellen der Brennstoffzufuhr wird vorteilhaft Luft verwendet.

Zur Überprüfung des Ionenbeschusses wird vorteilhaft eine metallene Prüffläche verwendet. Diese ist zweckmässigerweise aus einem für den Brennerbau geeigneten Material, z. B. Alloy 601, gefertigt. Die Prüffläche ist vorteilhaft in einen Brenner, z.B. in einen Verdampfer eingesetzt. Die Prüffläche kann die innere Oberfläche eines Flammbechers sein. Sie kann auch die Oberfläche eines Verdampfers sein. Sie kann insbesondere auch eine Oberfläche eines herkömmlichen Brenners sein.

Die Zündung erfolgt vorteilhaft durch einen Zündfunken. Dadurch können für die Zündung die Elektroden verwendet werden, die zur Errichtung eines Spannungsfelds ohnehin benötigt werden, und das Spannungsfeld ist in diesem Fall mit Sicherheit schon vorhanden, wenn das Plasma entsteht. Die Elektroden können mit einer Wechselspannung versorgt sein, oder eine konstante Potentialdifferenz aufweisen. Das Spannungsfeld kann zwischen zwei Elektroden aufgebaut werden, während die Prüffläche geerdet ist. Es kann auch zwischen einer Elektrode und der Prüffläche aufgebaut werden, so dass die Prüffläche eine Elektrode bildet. Diese die Prüffläche bildende Elektrode ist vorteilhaft aus Flachmaterial gefertigt.

In einer Heizanlage erfolgt eine Ölzufuhr vorschriftsmässig erst nach einer Vorzündzeit von 10 bis 15 Sekunden. Dies kann beim erfindungsgemässen Prüfverfahren ebenso gehandhabt werden. Wesentlich ist, dass das Plasma einem elektrischen Spannungsfeld ausgesetzt sind. Vorzugsweise ist das Spannungsfeld während dem Entstehen der Flamme bereits aufgebaut.

Es wurde gefunden, dass je länger in einer Heizanlage die Nachzündzeit eingestellt ist und der Zündfunken bzw. das Spannungsfeld gleichzeitig mit der Flamme aufrechterhalten bleibt, desto grösser ist die erodierende Wirkung eines Schadenbilder hervorrufenden Brennstoffes an benachbarten Metallteilen. Vorteilhaft wird daher beim Prüfverfahren in einer Sequenz während mehr als 35 sec., vorteilhaft bis zum Abstellen der Ölzufuhr, ein elektrisches Spannungsfeld aufrechterhalten.

Da die Erosion nahe der Elektrode oder Elektroden grösser ist als an entfernteren Stellen, sind beim Prüfverfahren die Elektroden oder ist die Elektrode vorteilhaft in einem Abstand von weniger als 50 mm, vorteilhaft in einem Abstand von weniger als 15 mm, oder gar in einem Abstand von 3 bis 7 mm von der metallenen Prüffläche, bzw. der Stelle der Prüffläche, nahe der die Ionisation des Brennstoffes geschieht. Die Spannung und der Abstand zwischen den Spannungspotentialen sind einander reziprok entsprechend derart zu wählen, dass ein möglichst grosses Volumen des Plasmas einer möglichst hohen Spannung ausgesetzt ist.

Je höher die Spannung des Spannungsfeldes ist, desto höher ist die Wahrscheinlichkeit, dass der Schadenbilder hervorrufende Brennstoff ein solches Schadenbild hervorruft. Daher wird das Plasma vorteilhaft einer Spannung von 2 mal 7500 V und mehr, vorzugsweise von wenigstens 2 mal 10'000 V, besonders bevorzugt von wenigstens 2 mal 15'000 Volt ausgesetzt. Es konnte jedoch festgestellt werden, dass einzelne Brennstoffe schon bei Spannungen von 200 V oder 230 V Schadenbilder hervorrufen.

Es konnte an Heizungsanlagen für schwefelarmes Heizöl extra leicht (Schwefelgehalt < 0.5 ppm) beobachtet werden, dass im Sprühschatten der Zündelektroden weniger Schadensbilder auftreten. Daher wird beim Prüfverfahren vorteilhaft eine im Flammenbereich angeordnete Metallfläche mit dem Heizöl besprüht oder gar benetzt. Damit die Ionisation des Brennstoffes möglichst lange nahe der Prüffläche eintritt, wird der Flammbecher oder eine andere Prüffläche vor dem Beginn einer neuen Sequenz vorteilhaft auf eine Temperatur unter 250°C, vorzugsweise unter 220°C, besonders bevorzugt unter 200°C abgekühlt. Eine Kühlung geschieht vorzugsweise durch Nachlüften während einigen Sekunden oder Minuten. Die Prüffläche ist zwecks Kühlung vorteilhaft axial im Frischluftstrom angeordnet. Vorteilhaft wird die Prüffläche während der gesamten Brenndauer gekühlt. Durch die Abkühlung zwischen den Sequenzen unter 250 Grad wird erreicht, dass bei der nächsten Sequenz die Prüffläche einen Temperaturbereich von 350 bis 550 oder 600 °C durchlaufen muss. Durch die Wahl der Sequenzlänge wird erreicht, dass die Prüffläche 550 bis 600 Grad erreicht, jedoch der Prozess nicht unnötig lange bei heisseren und daher weniger effizienten Temperaturen weitergeführt wird.

Je kürzer die Verbrennungsphase einer Sequenz dauert, desto weniger erhitzt sich die Umgebung der Flamme. Je länger das Prüfverfahren bei niedriger Temperatur des Flammbechers oder einer anderen Prüffläche abläuft, desto grösser ist der Zeitanteil, während dem die erodierende Metallfläche benetzt wird und die Bedingungen für die erodierende Wirkung des Brennstoffes optimal sind. Daher wird vorteilhaft eine Sequenz kurz gewählt, so dass sie weniger als 15 Minuten dauert, vorteilhaft weniger als 10 Minuten, besonders bevorzugt weniger als 5 Minuten. Dadurch kann der erosionswirksame Zeitanteil pro Stunde erhöht werden.

Mit einem solchen Verfahren kann eine Heizöl-Probe innerhalb von höchstens 30 bis 40 Betriebsstunden (Brenndauer) auf ihre erodierende Wirkung überprüft werden. Es ist zu erwarten, dass durch eine Optimierung des Prüfverfahrens diese Betriebszeit noch weiter verkürzt werden kann. Je nach Eigenschaften der Probe tritt eine erste von blossem Auge sichtbare Erosion bereits nach wenigen Betriebsstunden auf.

Eine erodierte oder nach z.B. 40 Stunden noch immer nicht erodierte Prüffläche wird zweckmässigerweise als Beleg der Heizölqualität einer Charge archiviert. Für jede zu prüfende Charge wird daher vorteilhaft eine neue Prüffläche in einer Prüfvorrichtung angeordnet. Diese Prüffläche kann vorgängig mit einer Kohlenschicht versehen werden. Es kann aber auch beim Starten des Prozesses die Verbrennung derart beeinflusst werden, dass sich eine Kohlenschicht auf der Prüffläche bildet.

Zur Prüfung von Heizöl oder anderen flüssigen Brennstoffen auf deren potentielle erosive Wirkung beim Verbrennen in Heizungsanlagen, insbesondere in Brennwert-Heizungsanlagen, d.h. bei Heizanlagen, in denen bei der Verbrennung entstehender Wasserdampf an der Kesselwandung kondensiert, wird erfindungsgemäss eine Vorrichtung verwendet, welche wenigstens folgendes aufweist: Einen Brennstoffbehälter für eine Brennstoffprobe, eine an den Brennstoffbehälter angeschlossenen Brennstoffpumpe, eine nach der Brennstoffpumpe angeordnete Düse zum Versprühen von Brennstoff, einen Spannungswandler, wenigstens eine mit dem Spannungswandler verbundene Elektrode, einen Ventilator für eine Frischluftzufuhr, eine Steuerung zur Steuerung der Brennstoffpumpe, des Spannungswandlers und des Ventilators, sowie eine einfach aus der Vorrichtung entfernbare und in die Vorrichtung einsetzbare Prüffläche. Bei einer solchen Vorrichtung kann ein Feuerungsraum oder Kessel gänzlich fehlen. Vorteilhaft ist jedoch ein Temperaturfühler zum Messen der Temperatur der Prüffläche vorhanden. Vorteilhaft ist ferner die Prüffläche metallisch und bevorzugt mit einem Kohlenstofffilm versehen.

Es wurde gefunden, dass eine Erosion der Prüffläche an Stellen, die Spuren von Filzstiften aufwiesen oder an Prüfflächen, die mit Werkzeugen aus Buntmetall bearbeitet wurden rascher auftrat. Es wird daraus geschlossen, dass Metalloxide, insbesondere Oxide von Nicht-Eisen-Metallen, an der Oberfläche der Prüffläche die Erosion begünstigen. Daher wird die Prüffläche vor dem Prüfverfahren vorteilhaft mit einem Metalloxid behandelt. Die Prüffläche kann auch mit Metallstaub behandelt werden, der dann bei der Verbrennung von Brennstoff oxidiert.

Die Steuerung ist vorteilhaft derart ausgelegt, dass sie eine Vielzahl der beschriebenen Sequenzen automatisch steuert.

Der Spannungswandler ist dabei vorteilhaft derart dimensioniert, dass er eine Spannung von zweimal mehr als 7'500 V, vorzugsweise zweimal mehr als 10'000 V, besonders bevorzugt von zweimal mehr als 15'000 Volt generiert. Herkömmliche Spannungswandler für Heizungsanlagen sind auf eine Spannung von zweimal 7500 Volt, eine Vorzündzeit von 12 sec. und eine Nachzündzeit von ca. 20 sec. ausgelegt. Für eine Prüfvorrichtung ist er jedoch vorteilhaft derart ausgelegt, dass er die Spannung über einen Zeitraum von mehr als 35 sec., vorzugsweise mehr als einer Minute, besonders bevorzugt praktisch unbegrenzt aufrecht erhalten kann. Die Spannung kann auch gegenüber der Erde als zweitem Potential aufgebaut werden. Es kann auch, allenfalls zusätzlich zur Spannung zwischen den Elektroden, eine konstante Potenzialdifferenz zwischen einer separaten Elektrode und der geerdeten oder unter Spannung gesetzten Prüffläche aufrecht erhalten werden. Es kann auch die Prüffläche gegenüber der Erde unter Spannung gesetzt werden. Durch eine dadurch erreichte Spannungsdifferenz zwischen der Prüffläche und anderen Teilen in der Umgebung des Plasmas kann der Ionenfluss gesteuert werden.

Die Elektrode ist, bzw. die Elektroden sind vorteilhaft in einem Abstand von weniger als 50 mm, vorteilhaft in einem Abstand von weniger als 15 mm von einer metallenen Prüffläche angeordnet. Vorteilhaft ist ein Temperaturfühler vorhanden, mit dem die Temperatur der Prüffläche überwacht werden kann.

Die Düse und die Prüffläche sind vorteilhaft so angeordnet und die Düse weist eine derartige Charakteristik auf, dass gewährleistet ist, dass der Brennstoff gegen die im Bereich der Flamme angeordnete Prüffläche spritzt und der Brennstoff die Prüffläche benetzt und nahe der Prüffläche in ein Plasma übergeht. Es kann eine erste Düse für eine die Flamme aufrechterhaltende Brennstoffzufuhr zur Flamme und eine zweite Düse zur Besprühung oder Benetzung der Prüffläche vorhanden sein. Die metallene Prüffläche ist vorteilhaft aus einem Edelstahl gefertigt, z.B. Alloy 601, DIN 2.4851, der gewährleistet, dass keine Hochtemperaturkorrosion auftritt. Dadurch ist ein auftretendes Schadensbild eindeutig der Erosion und der Qualität des geprüften Brennstoffs zuzuordnen.

Zur Vermeidung des Schadens kann nicht nur die Erosivität des Brennstoffs überprüft werden. Es können dem Brennstoff bei Bedarf, d.h. bei zu hoher Erosivität einer Charge, Antioxidantien beigemischt werden. Antioxidatien verhindern, wie auch ein Schwefelgehalt von über 0,4 bis 0,5%m/m, die Bildung von Carbonsäure. Ferner kann dem Heizöl ein Additiv zugesetzt werden, das gewährleistet, dass die elektrostatischen Ladungen bei der Verbrennung gering bleiben. Ein solches "Static Dissipator Additive" ist beispielsweise das bisher in Flugbezin verwendete "Stadis 450". Ob die Dosierung der Additive ausreicht wird vorteilhaft mittels des beschriebenen Prüfverfahrens überprüft.

Es wird auf Grund der Feststellung der erhöhten Erosivität des Brennstoffes bei Anwesenheit von Metalloxiden auch vermutet, dass zur Entschwefelung des Brennstoffes eingesetzte Metalloxide in Spuren im Brennstoff verbleiben und eine Erosion begünstigen. Daher kann zur Verhinderung der Erosion der Metalloxidgehalt der im Brennstoff reduziert oder deren Aggressivität durch eine Zugabe von Antioxidantien neutralisiert werden. Es wird daher angenommen, dass die Bemessung der Zugabemenge von Antioxidantien aufgrund einer Messung des Metalloxidgehalts des Brennstoffes vorgenommen werden kann.

Eine weitere Massnahme zur Verhinderung der beschriebenen Erosion besteht darin, den Brennstoff mit einer Glühzündung zu entzünden und so die elektrische Spannung im Brennraum möglichst gering zu halten. Ein vorteilhafter Blaubrenner ist daher mit einer Glühzündung, insbesondere mit einer Niedervolt-Glühzündung ausgerüstet. Dadurch wird verhindert, dass Ionen in grosser Zahl in einem Spannungsfeld beschleunigt werden, auf das Metall aufprallen und dadurch dessen Oberfläche erodieren.

Alle diese Massnahmen basieren auf der Erkenntnis, dass auf den die zu vermeidenden Schadensbilder verursachenden Prozess in zweierlei Hinsicht Einfluss genommen werden kann, nämlich:1. durch Verminderung einer elektrischen Spannung im Verbrennungsraum, und 2. durch die Verminderung der Bildung von Carbonsäure. Fehlt lediglich einer der genannten Faktoren, so ist das Schadensrisiko bereits wesentlich vermindert. Wie und weshalb die Brennstoffeigenschaften, die in jeder Charge wieder anders sein können, bei dem schadenden Prozess beteiligt sind, ist noch nicht erkennbar.

Kurzbeschreibung der Figuren:

Es zeigt:
- Fig. 1: eine teilweise geschnittene Seitenansicht eines Brenners für eine erfindungsgemässe Prüfvorrichtung,
- Fig. 2: Seitenansicht des Brennerkopfes,
- Fig. 3: Frontalansicht des Brennerkopfes,
- Fig. 4: eine geschnittenen Darstellung des Brennerkopfes,
- Fig. 5: eine schematische Darstellung einer Prüfvorrichtung.

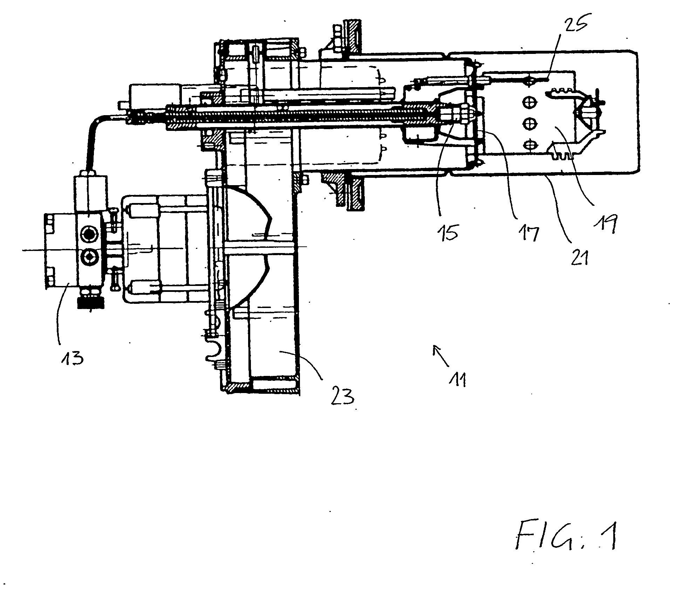

Der Brenner 11 gemäss Figur 1 weist eine Brennstoffpumpe 13 auf, mit der der Brennstoff aus einem nicht dargestellten Behälter der Brennstoffdüse 15 zugepumpt wird. Die Brennstoffdüse 15 ist mit ihrer Düsenöffnung etwa in der Ebene einer Stauscheibe 17 angeordnet. In der Stauscheibe 17 ist eine zentrale Öffnung vorhanden, durch die der Brennstoff in einen Verdampfer 19 gespritzt werden kann. Der Verdampfer 19 ist in einem Flammrohr 21 angeordnet.

Der Brenner 11 weist zudem einen Ventilator 23 auf. Mit dem Ventilator 23 kann Frischluft mit einem bestimmten Druck an die Stauscheibe 17 herangeführt werden. Die Frischluft tritt in der Folge in den Innenraum des Verdampfers 19 ein, wo sie sich mit verdampftem Brennstoff und mit rezirkulierten Verbrennungsgasen vermischt. Nicht dargestellt sind Temperaturfühler zur Überwachung der Temperatur des Brennstoffes und der Prüffläche. Nicht dargestellt ist ein Verbrennungsraum, in welchem die Flamme brennt.

Erkennbar sind zwei Zündelektroden 25 nahe der Vergaserwandung. Diese sind an einen nicht dargestellten Spannungswandler angeschlossen, der die Elektroden mit einer alternierenden Potentialdifferenz von zweimal 7500 Volt versorgt.

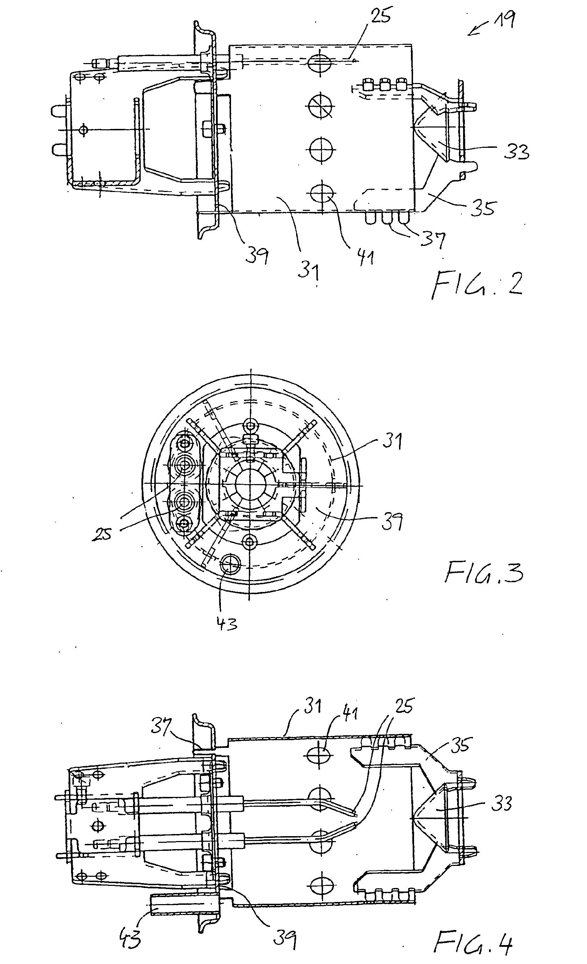

Der Verdampfer 19 ist in den Figuren 2 bis 4 dargestellt. Er weist ein Verdampferrohr 31 und einen Flammenteiler 33 auf. Der Flammenteiler 33 ist mit drei Beinen 35 am Verdampferrohr befestigt. Die Befestigung geschieht über Blechlappen 37, die an den Beinen 35 ausgeformt sind und in Schlitzen am Verdampferrohr 31 stecken. Diese eingesteckten Blechlappen 37 können um 20 bis 30° verdreht werden und sind dadurch mit dem Verdampferrohr 31 mechanisch fest verbunden. Ebenso ist das Verdampferrohr 31 an der Stauscheibe 39 mittels drei in Schlitzen steckenden und verdrehten Blechlappen 37 befestigt. Dadurch ist das Verdampferrohr 31 sehr einfach in die Stauscheibe 39 einsetzbar und wieder von der Stauscheibe 39 entfernbar.

Im Verdampferrohr sind Abgasrezirkulationsöffnungen 41 vorhanden. Der vom Verdampferrrohr umschlossene Raum dient der Durchmischung von Brennstoffdampf, Frischluft und rezirkulierten, sauerstoffarmen Verbrennungsgasen. Der durch die Düse 15 in diesen Raum eingespritze Brennstoff vergast in diesem Raum und brennt danach mit einer blauen Flamme mit relativ niedriger Temperatur und schadstoffarm.

Für die Zündung des Brennstoffes sind zwei Elektroden 25 vorhanden, die durch die Stauscheibe 39 hindurchgeführt sind. In der Stauscheibe 39 ist zudem eine Öffnung 43 für einen Temperaturfühler vorhanden.

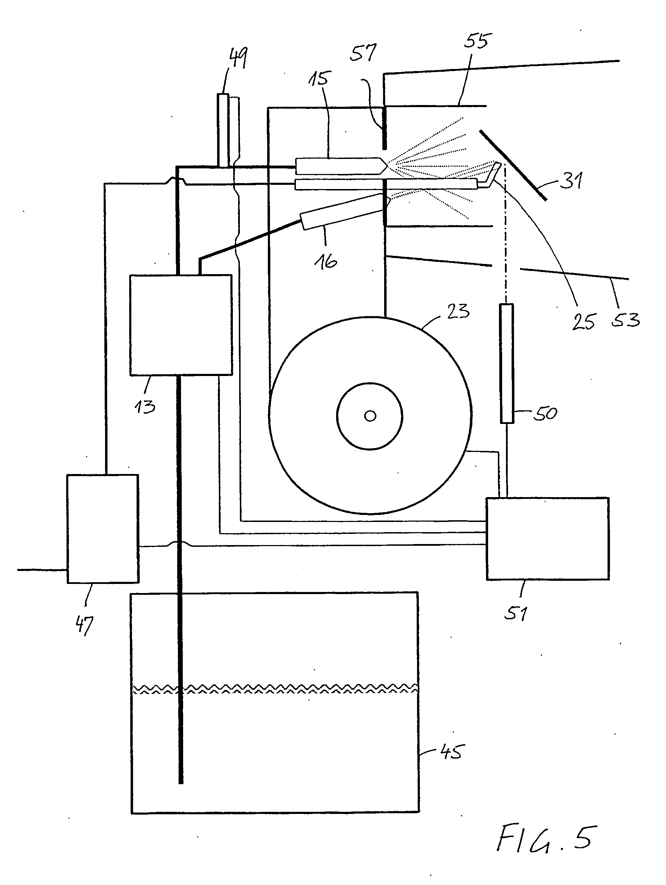

In der Figur 5 ist schematisch eine vorteilhafte Vorrichtung zur Prüfung von flüssigem Brennstoff, insbesondere Heizöl auf dessen potentielle erosive Wirkung beim Verbrennen in einer Heizungsanlage dargestellt. Diese weist einen Brennstoffbehälter 45 für eine Brennstoffprobe, eine an den Brennstoffbehälter 45 angeschlossene Brennstoffpumpe 13 und zwei nach der Brennstoffpumpe 13 angeordnete Düsen 15,16 auf. Die erste Düse 15 weist eine Kegelcharakteristik mit einem üblichen Winkel zum Versprühen von Brennstoff auf. Die zweite Düse 16 spritzt Brennstoff gezielt auf die Prüffläche 31. Sie weist dazu eine Charakteristik mit einem spitzen Kegelwinkel von wenigen Graden auf. Ferner ist ein Spannungswandler 47 und eine mit dem Spannungswandler 47 verbundene Elektrode 25 vorhanden. Ein Ventilator 23 für eine Frischluftzufuhr und eine Steuerung 51 zur Steuerung der Brennstoffpumpe 13, des Spannungswandlers 47 und des Ventilators 23 komplettieren den Prüfapparat. Weiter ist in der Vorrichtung zum Prüfen von flüssigem Brennstoff eine ersetzbare, metallene Prüffläche 31 im Bereich der Flamme angeordnet. Diese ist geerdet. Mit einem Temperaturfühler 49 kann die Öltemperatur überwacht werden. Mit einem Infrarotfühler 50 kann die Temperatur der Prüffläche 31 überwacht werden.

Für die Flamme ist ein Brennraum innerhalb einer Brennraumwandung 53 vorhanden, dessen Ausgestaltung von untergeordneter Bedeutung ist. Damit die Flamme ruhig brennt, ist ein Flammrohr 55 vorhanden. Die Frischluft wird durch das Gebläse 23 gegen eine Stauscheibe 57 geblasen. Diese besitzt zentral eine Luftöffnung, in der die erste Düse derart angeordnet ist, dass rings um die erste Düse 15 eine Ringöffnung vorliegt. Axial in der Strömungsrichtung der Luft ist die Prüffläche 31 angeordnet. In der Nähe und vor der Prüffläche 31 ist eine Elektrode 25 angeordnet, die mit beispielsweise 12'000 Volt Gleich- oder Wechselspannung aus dem Stromwandler 47 versehen werden kann. Die zweite Düse 16 ist auf die Prüffläche 31 gerichtet, und zwar so, dass der durch die zweite Düse eingespritzte Brennstoff etwa zentral auf die Prüffläche 31 auftrifft und diese benetzt. Somit ist diese Stelle der Prüffläche 31 durch den Luftstrom und den benetzenden Brennstoff gekühlt. Sie liegt in unmittelbarer Nähe zum bzw. im dem Spannungsfeld stark ausgesetzten Bereich des Plasmas. Diese Stelle wird auch durch den Infrarotfühler überwacht.

Die Steuerung 51 kann nach einem Zeitgeber regelmässig den Ventilator 23, die Ölpumpe 13, eventuell ein nicht dargestelltes Ventil zur Regelung der Düsen 15,16 und den Stromwandler 47 ansteuern. Sie kann diese aber auch aufgrund von Messewerten ansteuern. Als Messwert kommt insbesondere die Temperatur der Prüffläche 31 in Frage. So kann die Prüfverfahrens-Sequenz gestartet werden, sobald der Temperaturfühler der Prüffläche beispielsweise 250, 300, 350 oder 380 Grad misst. Die Sequenz kann beendet werden, wenn die Prüffläche 550, 600 oder 650 Grad misst.

Wesentlich an der Prüfungsvorrichtung ist ihre konstant gleichbleibende Ausbildung, die eine Vergleichbarkeit der Resultate gewährleistet.

In einer Prüfungsvorrichtung kann auch der Brennstoff auf eine Prüffläche aufgebracht werden, die elektrisch vorgewärmt ist. Dazu kann die Prüffläche aus einem Alloy-Streifen bestehen, der an eine Stromquelle angeschlossen ist und so direkt geheizt ist oder mit einer Heizwicklung indirekt geheizt ist. Der Brennstoff kann auf die Prüffläche aufgegossen, aufgetropft oder aufgesprüht werden. Die Verdunstung oder Verdampfung des Brennstoffes erfolgt dank der Wärmeenergiezufuhr zur Prüffläche. Dies erlaubt, die Prüffläche kontrolliert einen gewünschten Temperaturverlauf wiederholt durchlaufen zu lassen.

### Beispiele eines Ablaufes eines Prüfverfahrens:

Mit einem in den Figuren dargestellten Brenner 11 wird eine Brennstoffprobe geprüft. Es sind jedoch auch andere Prüfungsvorrichtungen möglich und sinnvoll. Zur Prüfung des Brennstoffes wird der Brennstoff mit der Düse 15 in das Vergaserrohr 31 gesprüht. Im Vergaserrohr wird der Brennstoff dank vorhandener Restwärme oder der mit den Elektroden zugeführten Energie vergast und am Zündfunken entflammt. Mit den Zündelektroden wird das Plasma einem elektrischen Spannungsfeld von zweimal 7500 Volt Wechselspannung ausgesetzt. Dank dem niedrigen Schwefelgehalt, der Gegenwart der mit dem Gebläse eingeblasenen sauerstoffreichen Frischluft und dem elektrischen Spannungsfeld sind die Verbrennungsbedingungen derart, dass am Vergaserrohr 31 sehr rasch erodierte Stellen entstehen.

In der Versuchsphase werden Zyklen von 15 Minuten gewählt, um eine Reproduzierbarkeit der Versuchsreihe zu gewährleisten. Der Versuch wird mit dem Brenner gemäss Figuren 1 bis 4 ausgeführt. Es wird während etwa 15 Sekunden vorgezündet. Danach wird bei laufender Frischluftzufuhr das zu prüfende Heizöl eingespritzt. Es benetzt die innere Oberfläche des Vergaserrohrs. Sofort entzündet es sich am Zündfunken. Das entstehende Plasma wird während der gesamten Brenndauer dem elektrischen Spannungsfeld zwischen den Elektroden ausgesetzt. Während 5 Minuten wird die Flamme so aufrechterhalten und einer Potenzialdifferenz zwischen der Prüffläche und der Elektrode ausgesetzt. Danach wird die Ölzufuhr abgestellt und nachgelüftet. Die Nachlüftzeit beträgt 10 Minuten. Dann beginnt der Zyklus von vorne.

Mit diesem Zyklus sind 20 Minuten Brenndauer innerhalb einer Stunde der Versuchszeit erosionswirksam. Nach spätestens 120 Stunden oder 5 Tagen Versuchszeit liegt ein zuverlässiges Prüfungsergebnis vor. Das Verdampferrohr 31 wird ausgebaut und überprüft. Das Ausmass der Erosion gibt Aufschluss über die erosive Wirkung des Heizöls der geprüften Charge in Heizungsanlagen. Das Verdampferrohr wird zum Beleg der Brennstoffqualität beschriftet und archiviert.

Es ist zu erwarten, dass mit kürzeren Zyklen von 3 Minuten Brenndauer und 6 Minuten Nachlüftzeit die Versuchszeit verkürzt werden kann. Dies ist darauf zurückzuführen, dass die Erosion in der kühleren Startphase stärker auftritt, solange der Brennstoff die Prüffläche noch flüssig erreicht. Durch geeignete Massnahmen zur Verkürzung der Nachlüftzeit kann die Versuchszeit zusätzlich verkürzt werden.

Eine bevorzugte Sequenz weist 2 Minuten Brenndauer auf, die von 3 Minuten Nachlüften gefolgt ist. Die Vorzündzeit übergreift die Nachlüftzeit und ist kurz, beispielsweise 2 Sekunden. Danach setzt die Brennstoffpumpe für 2 Minuten ein, während denen die geerdete Prüffläche besprüht wird. Während der gesamten Zeit der Ölzufuhr wird eine Spannung von ca. 15'000 Volt gegenüber der Erde auf die einzige Elektrode gegeben. Die Erosivität des Brennstoffes kann bei dieser Prüfungsanlage nach ca. 3 Tagen der Versuchsdauer beurteilt werden.

## Patentansprüche

1. Verfahren zur Vermeidung von Erosionsschäden in Heizungsanlagen, **dadurch gekennzeichnet, dass** flüssiger, Brennstoff mit einem Massenanteil an Schwefel von höchstens 500ppm, insbesondere Dieselöl oder Heizöl, auf dessen potentielle erosive Wirkung beim Verbrennen in Heizungsanlagen geprüft wird.

2. Verfahren nach Anspruch 1, **dadurch gekennzeichnet, dass** die Prüfung des Brennstoffs chargenweise erfolgt.

3. Verfahren nach Anspruch 1 oder 2, **dadurch gekennzeichnet, dass** zur Prüfung eine Prüffläche vorgesehen wird, nahe der Prüffläche der Brennstoff vernebelt, der Brennstoffnebel einem elektrischen Spannungsfeld ausgesetzt und bei aufrechterhaltenem Spannungsfeld entflammt wird.

4. Verfahren nach Anspruch 3, **dadurch gekennzeichnet, dass** das durch das Entflammen des Brennstoffs entstandene Plasma einem elektrischen Spannungsfeld ausgesetzt wird.

5. Verfahren nach Anspruch 3 oder 4, **dadurch gekennzeichnet, dass** die Prüffläche aus Metall besteht.

6. Verfahren nach Anspruch 5, **dadurch gekennzeichnet, dass** das Metall ein Alloy-Stahl ist.

7. Verfahren nach Anspruch 5 oder 6, **dadurch gekennzeichnet, dass** die Prüffläche durch wenigstens eine Elektrode gebildet ist.

8. Verfahren nach einem der Ansprüche 1 bis 7, **gekennzeichnet durch** eine mehrfache Wiederholung einer Sequenz mit folgenden Verfahrensmerkmalen:
- Vernebeln von flüssigem Brennstoff,
- den Brennstoffnebel einem elektrischen Spannungsfeld aussetzen,
- Zünden des Brennstoffes, um ein Plasma zu erreichen,
- das Plasma einem elektrischen Spannungsfeld aussetzen,
- Abstellen der Brennstoffzufuhr,

9. Verfahren nach Anspruch 8 in Verbindung mit Anspruch 3, **dadurch gekennzeichnet, dass** die Prüffläche anschliessend an diese Sequenz gekühlt wird.

10. Verfahren nach Anspruch 8 oder 9, **dadurch gekennzeichnet, dass** in einer Sequenz während mehr als 35 sec., vorteilhaft während der gesamten Zeit des Sprühens, das Plasma einem elektrischen Spannungsfeld ausgesetzt wird.

11. Verfahren nach einem der Ansprüche 3 bis 7, 9 oder 10, **dadurch gekennzeichnet, dass** eine Potentialdifferenz in einem mittleren Abstand von weniger als 50 mm, vorteilhaft in einem Abstand von weniger als 15 mm, besonders bevorzugt in einem Abstand von 3 bis 7 mm von der metallenen Prüffläche (31) aufrechterhalten wird.

12. Verfahren nach einem der Ansprüche 1 bis 11, **dadurch gekennzeichnet, dass** der Brennstoff einer Spannung von über zweimal 7500 V, vorzugsweise von über zweimal 10'000 V, besonders bevorzugt von zweimal 15'000 Volt und mehr ausgesetzt wird.

13. Verfahren nach einem der Ansprüche 3 bis 12, **dadurch gekennzeichnet, dass** die Prüffläche (31) mit dem Brennstoff benetzt wird.

14. Verfahren nach einem der Ansprüche 3 bis 13, **dadurch gekennzeichnet, dass** die Prüffläche vor oder zu Beginn des Prüfverfahrens mit einem Kohlenstofffilm versehen wird.

15. Verfahren nach einem der Ansprüche 3 bis 13, **dadurch gekennzeichnet, dass** die Prüffläche zumindest partiell mit Metalloxid eines Nicht-Eisen-Metalls behandelt ist.

16. Verfahren nach einem der Ansprüche 1 bis 15 in Verbindung mit Anspruch 8, **dadurch gekennzeichnet, dass** die Prüffläche bei jeder Sequenz einen Temperaturbereich von 350 bis 650 °C, bevorzugt einen Temperaturbereich von 370 bis 550 °C durchläuft.

17. Verfahren nach einem der Ansprüche 1 bis 16 in Verbindung mit Anspruch 8, **dadurch gekennzeichnet, dass** eine Sequenz maximal 15 Minuten dauert, vorteilhaft weniger als 10 Minuten, besonders bevorzugt weniger als 5 Minuten.

18. Verfahren nach einem der Ansprüche 1 bis 17 in Verbindung mit Anspruch 9, **dadurch gekennzeichnet, dass** die Prüffläche (31) auf eine Temperatur unter 250° abgekühlt wird, vorzugsweise unter 220°, besonders bevorzugt unter 200°.

19. Verfahren nach einem der Ansprüche 1 bis 17, **dadurch gekennzeichnet, dass** die Prüffläche (31) während der Brenndauer gekühlt wird.

20. Verfahren nach Anspruch 19, **dadurch gekennzeichnet, dass** der Brennstoff gekühlt wird auf unter 15°C, vorteilhaft auf maximal 10°C.

21. Verfahren nach Anspruch 19 oder 20, **dadurch gekennzeichnet, dass** dem Brennstoff ein Fliessverbesserer zugesetzt wird und der Brennstoff auf eine Temperatur unter 0°C, vorzugsweise unter minus 5°C, besonders bevorzugt auf etwa minus 10°C abgekühlt wird.

22. Verfahren nach einem der Ansprüche 1 bis 21, **dadurch gekennzeichnet, dass** dem Brennstoff, insbesondere Heizöl, ein Antioxidans zugesetzt wird.

23. Verfahren nach einem der Ansprüche 1 bis 22, **dadurch gekennzeichnet, dass** dem Brennstoff; ein Additiv zum Verhindern oder Abbauen von bei der Verbrennung des Brennstoffs auftretenden elektrostatischen Aufladungen (Static Dissipator Additive) zugesetzt wird.

24. Verfahren nach Anspruch 22 oder 23, **dadurch gekennzeichnet, dass** die bei einer Verbrennung in einer Heizungsanlage potientell auftretende erosive Wirkung des mit einem Antioxidans und /oder einem Additiv zur Verhinderung oder zum Abbauen von elektrostatischen Aufladungen versehenen Brennstoffs mittels einem Verfahren gemäss einem der Patentansprüche 1 bis 20 überprüft wird.

25. Verfahren nach einem der Ansprüche 1 oder 2, **dadurch gekennzeichnet, dass** der Metalloxidgehalt des Brennstoffes bestimmt wird, und dem Metalloxidgehalt entsprechend dem Brennstoff ein Antioxidans zugesetzt wird.

26. Vorrichtung zur Prüfung von flüssigem Brennstoff mit einem Massenanteil an Schwefel von höchstens 500ppm nach einem Verfahren gemäss einem der vorangehenden Ansprüche, insbesondere zur Prüfung von Heizöl auf dessen potentielle erosive Wirkung beim Verbrennen in einer Heizungsanlage, mit einem Brennstoffbehälter (45) für eine Brennstoffprobe, einer an den Brennstoffbehälter angeschlossenen Brennstoffpumpe (13), einer nach der Brennstoffpumpe (13) angeordneten Düse (15) zum Versprühen von Brennstoff, einem Spannungswandler (47), wenigstens einer mit dem Spannungswandler (47) verbunden Elektrode (25), einem Ventilator (23) für eine Frischluftzufuhr, einer Steuerung (51) zur Steuerung der Brennstoffpumpe (13), des Spannungswandlers (47) und des Ventilators (23), und einer ersetzbaren, Prüffläche (31), **dadurch gekennzeichnet, dass** ein Temperaturfühler (50) zum Überwachen der Temperatur der Prüffläche (31) vorhanden ist.

27. Vorrichtung nach Anspruch 26, **dadurch gekennzeichnet, dass** der Spannungswandler (47) eine Spannung von über zweimal 7'500 V, vorteilhaft über zweimal 10'000 V, besonders bevorzugt von über zweimal 15'000 Volt generiert.

28. Vorrichtung nach Anspruch 26 oder 27, **dadurch gekennzeichnet, dass** der Spannungswandler (47) derart ausgelegt ist, dass er das Spannungspotential über einen Zeitraum von mehr als 35 sec, vorzugsweise mehr als eine Minute, besonders bevorzugt praktisch unbegrenzt aufrecht erhalten kann.

29. Vorrichtung nach einem der Ansprüche 26 bis 28, **dadurch gekennzeichnet, dass** eine Elektrode (25) in einem Abstand von weniger als 50 mm, vorteilhaft in einem Abstand von weniger als 15 mm von einer metallenen Prüffläche (31) angeordnet ist.

30. Vorrichtung nach einem der Ansprüche 26 bis 28, **dadurch gekennzeichnet, dass** eine Elektrode die Prüffläche bildet.

31. Vorrichtung nach einem der Ansprüche 26 bis 30, **dadurch gekennzeichnet, dass** ein Temperaturfühler (49) vorgesehen ist, mit dem die Temperatur des einzuspritzenden Brennstoffes überwacht werden kann.

32. Vorrichtung nach einem der Ansprüche 26 bis 31, **dadurch gekennzeichnet, dass** eine Düse (15,16) und die Prüffläche (31) so angeordnet sind und die Düse (15,16) eine solche Charakteristik aufweist, dass gewährleistet ist, dass das Heizöl gegen die Prüffläche (31) spritzt.

33. Vorrichtung nach Anspruch 32, **dadurch gekennzeichnet, dass** eine erste Düse (15) für die Brennstoffzufuhr zur Flammenbasis und eine zweite Düse (16) zur Brennstoffzufuhr in das Spannungsfeld vorhanden ist.

34. Vorrichtung nach einem der Ansprüche 26 bis 33, **dadurch gekennzeichnet, dass** die Prüffläche (31) aus Metall gefertigt ist, vorteilhaft aus einem Edelstahl, z.B. Alloy 601, DIN 2.4851, der gewährleistet, dass keine Hochtemperaturkorrosion auftritt.

## Claims

1. Method for avoiding erosion damage in heating installations, **characterized in that** the potential erosive effect of liquid combustible with a sulfur mass fraction of 500 ppm at the most, in particular diesel oil or fuel oil, is tested when burning in heating installations.

2. Method according to claim 1, **characterized in that** the test of the combustible takes place for batch quantities.

3. Method according to claim 1 or 2, **characterized in that** for testing a test surface is provided, the combustible is atomized close to the test surface, the combustible mist is exposed to an electrical field and is ignited while the electrical field is maintained.

4. Method according to claim 3, **characterized in that** the plasma resulting from the ignition of the combustible is exposed to an electrical field.

5. Method according to claim 3 or 4, **characterized in that** the test surface is made of metal.

6. Method according to claim 5, **characterized in that** the metal is an alloy steel.

7. Method according to claim 5 or 6, **characterized in that** the test surface is formed by at least one electrode.

8. Method according to any of the claims 1 to 7, **characterized by** a multiple repetition of a sequence with the following method characteristics:
- atomizing of liquid combustible,
- exposure of the combustible mist to an electrical field,
- ignition of the combustible in order to obtain a plasma,
- exposure of the plasma to an electrical field,
- cutting off of the combustible supply.

9. Method according to claim 8 in relation with claim 3, **characterized in that** the test surface is cooled after this sequence.

10. Method according to claim 8 or 9, **characterized in that** the plasma is exposed to an electrical field in a sequence during more than 35 seconds, advantageously during the whole time of the atomizing.

11. Method according to any of the claims 3 to 7, 9 or 10, **characterized in that** a potential difference is maintained at a mean distance of less than 50 mm, advantageously at a distance of less than 15 mm, particularly preferably in a distance of 3 to 7 mm from the metal test surface (31).

12. Method according to any of the claims 1 to 11, **characterized in that** the combustible is exposed to a voltage of over twice 7500 V, preferably of over twice 10000 V, particularly preferably of over twice 15000 V and more.

13. Method according to any of the claims 3 to 12, **characterized in that** the test surface (31) is moistened with the combustible.

14. Method according to any of the claims 3 to 13, **characterized in that** the test surface is provided before or at the beginning of the test method with a carbon film.

15. Method according to any of the claims 3 to 13, **characterized in that** the test surface is treated at least partially with metal oxide of a nonferrous metal.

16. Method according to any of the claims 1 to 15 in relation with claim 8, **characterized in that** the test surface at each sequence passes through a temperature range of 350 to 650°C, preferably through a temperature range of 370 to 550° C.

17. Method according to any of the claims 1 to 16 in relation with claim 8, **characterized in that** a sequence lasts maximally 15 minutes, advantageously less than 10 minutes, particularly preferably less than 5 minutes.

18. Method according to any of the claims 1 to 17 in relation with claim 9, **characterized in that** the test surface (31) is cooled to a temperature below 250°, preferably below 220°, particularly preferaly below 200°.

19. Method according to any of the claims 1 to 17, **characterized in that** the test surface (31) is cooled during the combustion time.

20. Method according to claim 19, **characterized in that** the combustible is cooled to below 15° C, advantageously to maximum 10° C.

21. Method according to claim 19 or 20, **characterized in that** a flow improver is added to the combustible and the combustible is cooled down to a temperature below 0° C, preferably under minus 5° C, particularly preferably to approximately minus 10° C.

22. Method according to any of the claims 1 to 21, **characterized in that** an antioxidant agent is added to the combustible, in particular to the fuel oil.

23. Method according to any of the claims 1 to 22, **characterized in that** an additive for avoiding or reducing electrostatic charges occuring during the combustion of the combustible (static disspator additive) is added to the combustible.

24. Method according to claim 22 or 23, **characterized in that** the erosive effect of the combustible provided with an antioxidant agent and/or an additive for avoiding or reducing electrostatic charges potentially occuring during a combustion in a heating installation is tested by means of a method according to any of the patent claims 1 to 20.

25. Method according to any of the claims 1 or 2, **characterized in that** the metallic oxide content of the combustible is determined and that an antioxidant agent is added to the combustible according to its metallic oxide content.

26. Device for testing liquid combustible with a sulfur mass fraction of 500 ppm at the most according to a method according to any of the preceding claims, in particular for testing the potential erosive effect of heating oil during the combustion in a heating installation, with a combustible container (45) for a combustible sample, a combustible pump (13) connected to the combustible container, a nozzle (15) placed after the combustible pump (13) for atomizing combustible, a voltage transformer (47), at least one electrode (25) connected with the voltage transformer (47), a fan (23) for a fresh air supply, a control (51) for controlling the combustible pump (13), the voltage transformer (47) and the fan (23) and a replaceable test surface (31), **characterized in that** a temperature probe (50) exists for the temperature supervision of the test surface (31).

27. Device according to claim 26, **characterized in that** the voltage transformer (47) generates a voltage of over twice 7500 V, advantageously of over twice 10000 V, particularly preferably of over twice 15000 V.

28. Device according to claim 26 or 27, **characterized in that** the voltage transformer (47) is designed such that it can maintain the voltage potential over a period of more than 35 seconds, preferably of more than one minute, particularly preferably pratically unlimited.

29. Device according to any of the claims 26 to 28, **characterized in that** an electrode (25) is placed at a distance of less than 50 mm, advantageously at a distance of less than 15 mm of a metallic test surface (31).

30. Device according to any of the claims 26 to 28, **characterized in that** an electrode forms the test surface.

31. Device according to any of the claims 26 to 30, **characterized in that** a temperature probe (49) is provided with which the temperature of the combustible to be injected can be supervised.

32. Device according to any of the claims 26 to 31, **characterized in that** a nozzle (15, 16) and the test surface (31) are placed in such a manner and the nozzle (15, 16) has such a characteristic curve that it is guaranteed that the fuel oil splashes against the test surface (31).

33. Device according to claim 32, **characterized in that** there is a first nozzle (15) for the combustible supply to the flame base and a second nozzle (16) for the combustible supply into the electrical field.

34. Device according to any of the claims 26 to 33, **characterized in that** the test surface (31) is made of metal, advantageously of a stainless steel, for example alloy 601, DIN 24851 which guarantees that no high temperature corrosion takes place.

## Revendications

1. Procédé pour éviter des dégâts d'érosion dans des installations de chauffage, **caractérisé en ce que** du combustible liquide avec un rapport massique de soufre de 500 ppm maximum, en particulier du gazole ou du fuel, est testé quant à son effet érosif potentiel lors de la combustion dans des installations de chauffage.

2. Procédé selon la revendication 1, **caractérisé en ce que** le test du combustible est effectué par charges successives.

3. Procédé selon la revendication 1 ou 2, **caractérisé en ce que** pour le test il est prévu une surface de test, que le combustible est atomisé près de la surface de test, que le brouillard de combustible est soumis à un champ électrique et est enflammé, le champ électrique étant maintenu.

4. Procédé selon la revendication 3, **caractérisé en ce que** le plasma provenant de l'inflammation du combustible est soumis à un champ électrique.

5. Procédé selon la revendication 3 ou 4, **caractérisé en ce que** la surface de test est en métal.

6. Procédé selon la revendication 5, **caractérisé en ce que** le métal est un acier d'alliage.

7. Procédé selon la revendication 5 ou 6, **caractérisé en ce que** la surface de test est formée par au moins une électrode.

8. Procédé selon l'une des revendications 1 à 7, **caractérisé par** une répétition multiple d'une séquence avec les caractéristiques de procédé suivantes :
- nébulisation de combustible liquide,
- soumission du brouillard de combustible à un champ électrique,
- ignition du combustible pour obtenir un plasma,
- soumission du plasma à un champ électrique,
- arrêt de l'alimentation en combustible.

9. Procédé selon la revendication 8 en relation avec la revendication 3, **caractérisé en ce que** la surface de test est refroidie après cette séquence.

10. Procédé selon la revendication 8 ou 9, **caractérisé en ce que** le plasma est soumis à un champ électrique dans une séquence pendant plus de 35 secondes, de manière avantageuse pendant tout le temps de la pulvérisation.

11. Procédé selon l'une des revendications 3 à 7, 9 ou 10, **caractérisé en ce qu'**une différence de potentiel est maintenue à une distance moyenne de moins de 50 mm, de manière avantageuse à une distance de moins de 15 mm, de manière particulièrement préférée à une distance de 3 à 7 mm de la surface de test métallique (31).

12. Procédé selon l'une des revendications 1 à 11, **caractérisé en ce que** le combustible est soumis à une tension de plus de deux fois 7500 V, de préférence de plus de deux fois 10000 V, de manière particulièrement préférée de deux fois 15000 V et plus.

13. Procédé selon l'une des revendications 3 à 12, **caractérisé en ce que** la surface de test (31) est humidifiée avec le combustible.

14. Procédé selon l'une des revendications 3 à 13, **caractérisé en ce que** la surface de test est pourvue avant ou au début du procédé de test d'un film de carbone.

15. Procédé selon l'une des revendications 3 à 13, **caractérisé en ce que** la surface de test est traitée au moins partiellement avec de l'oxyde métallique d'un métal non ferreux.

16. Procédé selon l'une des revendications 1 à 15 en relation avec la revendication 8, **caractérisé en ce que** la surface de test traverse, à chaque séquence, une plage de température de 350 à 650° C, de préférence une plage de température de 370 à 550° C.

17. Procédé selon l'une des revendications 1 à 16 en relation avec la revendication 8, **caractérisé en ce qu'**une séquence dure 15 minutes au maximum, de manière avantageuse moins de 10 minutes, de manière particulièrement préférée moins de 5 minutes.

18. Procédé selon l'une des revendications 1 à 17 en relation avec la revendication 9, **caractérisé en ce que** la surface de test (31) est refroidie à une température inférieure à 250°, de préférence inférieure à 220°, de manière particulièrement préférée inférieure à 200°.

19. Procédé selon l'une des revendications 1 à 17, **caractérisé en ce que** la surface de test (31) est refroidie pendant la durée de combustion.

20. Procédé selon la revendication 19, **caractérisé en ce que** le combustible est refroidi en dessous de 15° C, de manière avantageuse à 10° C maximum.

21. Procédé selon la revendication 19 ou 20, **caractérisé en ce qu'**un améliorant d'écoulement est ajouté au combustible et que le combustible est refroidi à une température inférieure à 0° C, de préférence en dessous de moins 5° C, de manière particulièrement préférée à environ moins 10° C.

22. Procédé selon l'une des revendications 1 à 21, **caractérisé en ce qu'**un antioxydant est ajouté au combustible, en particulier au fuel.

23. Procédé selon l'une des revendications 1 à 22, **caractérisé en ce qu'**un additif pour empêcher ou diminuer des charges électrostatiques qui interviennent lors de la combustion du combustible (static dissipator additive) est ajouté au combustible.

24. Procédé selon la revendication 22 ou 23, **caractérisé en ce que** l'effet érosif du combustible pourvu d'un antioxydant et/ou d'un additif pour empêcher ou pour diminuer les charges électrostatiques, effet qui intervient potentiellement lors d'une combustion dans une installation de chauffage, est testé au moyen d'un procédé selon l'une des revendications 1 à 20.

25. Procédé selon l'une des revendications 1 ou 2, **caractérisé en ce que** la teneur en oxyde métallique du combustible est définie et qu'un antioxydant est ajouté au combustible selon sa teneur en oxyde métallique.

26. Dispositif pour tester du combustible liquide avec un rapport massique de soufre de 500 ppm maximum selon un procédé selon l'une des revendications précédentes, en particulier pour tester du fuel, quant à son effet érosif potentiel lors de la combustion dans une installation de chauffage, avec un réservoir de combustible (45) pour un échantillon de combustible, une pompe de combustible (13) raccordée au réservoir de combustible, une tuyère (15) placée après la pompe à combustible (13) pour pulvériser du combustible, un transformateur de tension (47), au moins une électrode (25) reliée au transformateur de tension (47), un ventilateur (23) pour une alimentation en air frais, une commande (51) pour la commande de la pompe de combustible (13), du transformateur de tension (47) et du ventilateur (23), et une surface de test remplaçable (31), **caractérisé en ce qu'**une sonde de température (50) existe pour surveiller la température de la surface de test (31).

27. Dispositif selon la revendication 26, **caractérisé en ce que** le transformateur de tension (47) génère une tension de plus de deux fois 7500 V, de manière avantageuse de plus de deux fois 10000 V, de manière particulièrement préférée de plus de deux fois 15000 V.

28. Dispositif selon la revendication 26 ou 27, **caractérisé en ce que** le transformateur de tension (47) est conçu de telle manière qu'il peut maintenir le potentiel de tension pendant une période de plus de 35 secondes, de préférence de plus d'une minute, de manière particulièrement préférée de manière pratiquement illimitée.

29. Dispositif selon l'une des revendications 26 à 28, **caractérisé en ce qu'**une électrode (25) est placée à un écart de moins de 50 mm, de manière avantageuse à un écart de moins de 15 mm d'une surface de test en métal (31).

30. Dispositif selon l'une des revendications 26 à 28, **caractérisé en ce qu'**une électrode forme la surface de test.

31. Dispositif selon l'une des revendications 26 à 30, **caractérisé en ce qu'**une sonde de température (49) est prévue avec laquelle la température du combustible à injecter peut être surveillée.

32. Dispositif selon l'une des revendications 26 à 31, **caractérisé en ce qu'**une tuyère (15, 16) et la surface de test (31) sont placées de telle manière et la tuyère (15, 16) présente une telle caractéristique qu'il est garanti que le fuel jaillilt contre la surface de test (31).

33. Dispositif selon la revendication 32, **caractérisé en ce qu'**une première tuyère (15) existe pour l'alimentation en combustible vers la base de la flamme et une seconde tuyère (16) existe pour l'alimentation en combustible dans le champ électrique.

34. Dispositif selon l'une des revendications 26 à 33, **caractérisé en ce que** la surface de test (31) est fabriquée en métal, de manière avantageuse en acier inoxydable, par exemple en alliage 601, DIN 24851 qui garantit qu'il n'y ait pas de corrosion à haute température.
